# EUROPÄISCHE PATENTSCHRIFT

(11) **EP 0 718 313 B1**
(45) Veröffentlichungstag und Bekanntmachung des Hinweises auf die Patenterteilung: **11.10.2000**
(21) Anmeldenummer: 95810786.4
(22) Anmeldetag: 13.12.1995
(51) Int. Cl.: C08B 37/08, A61K 7/48

(54) **N-cyanomethylierte Chitosane und deren Hydrolyseprodukte**
N-cyanomethylated chitosan and hydrolysis products thereof
Chitosane N-cyanométhylée et produits d'hydrolyse

(30) Priorität: 22.12.1994 CH 389994
(43) Veröffentlichungstag der Anmeldung: 26.06.1996
(73) Patentinhaber: Ciba Specialty Chemicals Holding Inc., 4057 Basel (CH)
(72) Erfinder: Haase, Jürg, CH-4126 Bettingen (CH); Kuhn, Martin, Dr., CH-4143 Dornach (CH)

(56) Entgegenhaltungen:
- DE-B- 1 112 081
- CARBOHYDRATE POLYMERS, Bd. 24, Nr. 3, 1994 BARKING GB, Seiten 209-214, XP 000469049 PHAM LE DUNG ET AL. 'Water soluble derivatives obtained by controlled chemical modifications of chitosan'
- CARBOHYDRATE POLYMERS, Bd. 8, Nr. 1, 1988 BARKING GB, Seiten 1-21, RICCARDO A.A. MUZZARELLI 'Carboxymethylated Chitins and Chitosans'
- CARBOHYDRATE RESEARCH, Bd. 107, Nr. 2, 1.September 1982 AMSTERDAM NL, Seiten 199-214, R. A. A. MUZZARELLI ET AL. 'N-(Carboxymethylidene)Chitosans and N-(Carboxymethyl)Chitosans.'

## Beschreibung

Die vorliegende Erfindung betrifft N-cyanomethylierte Chitosane, die Herstellung dieser Verbindungen, die Verwendung dieser Verbindungen als Trennmembranen, die Herstellung der hydrolysierten N-cyanomethylierten Chitosane, die Verwendung dieser hydrolysierten Verbindungen als Verdikkungsmittel, Repulsionsmittel, Sequestriermittel, Komplexbildner, Inkrustierungsinhibitor, Stabilisatoren für Bleichmittel sowie als Feuchthaltemittel in kosmetischen Präparaten.

In Carbohydrate Polymers, 24(3), 209-214 (1994) wird die Herstellung von N-carboxymethylierten Chitosanen offenbart, wobei N-mono- sowie N-disubstituierte Verbindungen in verschiedenen Verhältnissen hergestellt werden.

In Carbohydrate Polymers, 8(1), 1-21 (1988) wird die Herstellung und die Verwendung von carboxymethylierten Chitosanen offenbart.

In Carbohydrate Research 107(2), 199-214 (1982) wird offenbart, dass die funktionellen N-Carboxymethyl-Gruppen (*N*-CM) für verschiedene Eigenschaften dieser Polymerverbindungen verantwortlich sind, wie z.B. Komplexbildnereigenschaften.

DE-B-1,112,081 offenbart ein Verfahren zur Herstellung von Aminoacetonitrilen aus Formaldehyd, Blausäure und Ammoniak.

Die erfindungsgemässen N-cyanomethylierten Chitosane weisen wiederkehrende Einheiten der Formel 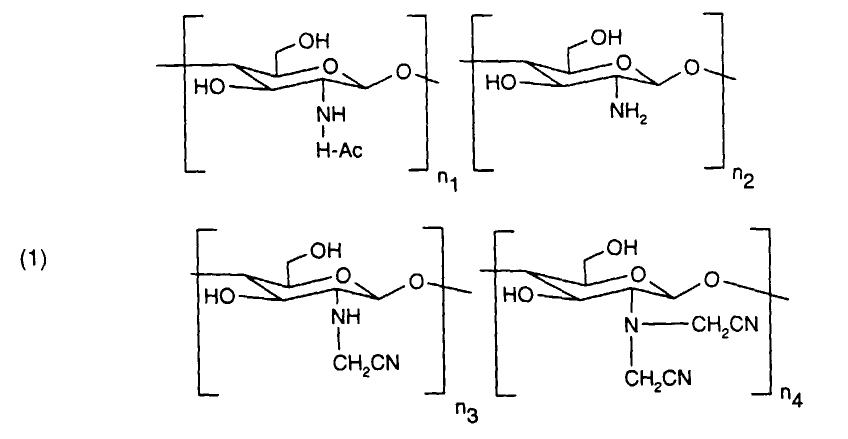 auf, worin
- n₁: 0 bis 0,6, vorzugsweise 0,05 bis 0,20; bezogen auf x:
- n₂: 0 bis 0,20, vorzugsweise 0,05 bis 0,10; bezogen auf x;
- (n₃ + n₄): 0,4 bis 1,0, vorzugsweise 0,8 bis 0,95; bezogen auf x;
bedeuten,
wobei x den Polymerisationsgrad von Glucosamin-Resten des Chitosans darstellt und eine Zahl von 5 bis 10000 bedeutet.

Die erfindungsgemässen N-cyanomethylierten Chitosane leiten sich vom Chitosan ab, das durch Deacetylierung von Chitin, einem natürlich vorkommenden Poly-(N-Acetylglucosamin), erhalten wird. Mitumfasst sind hierbei Chitosane, die in den üblichen Chitin-Deacetylierungsprozessen bis auf oligomere Fragmente abgebaut werden.

Das Chitosan ist im neutralen und alkalischen Medium unlöslich, es bildet jedoch aufgrund seiner chemischen Struktur im sauren Medium mit bestimmten organischen und anorganischen Säuren lösliche Salze.

Die Herstellung der erfindungsgemässen N-cyanomethylierten Chitosane erfolgt erfindungsgemäss in einer der Strecker-Synthese analogen Reaktion durch Umsetzung von Chitosan mit Formaldehyd und Cyanwasserstoff. Der gesamte Reaktionsablauf, einschliesslich der Chitin-Deacetylierung lässt sich folgendermassen darstellen: 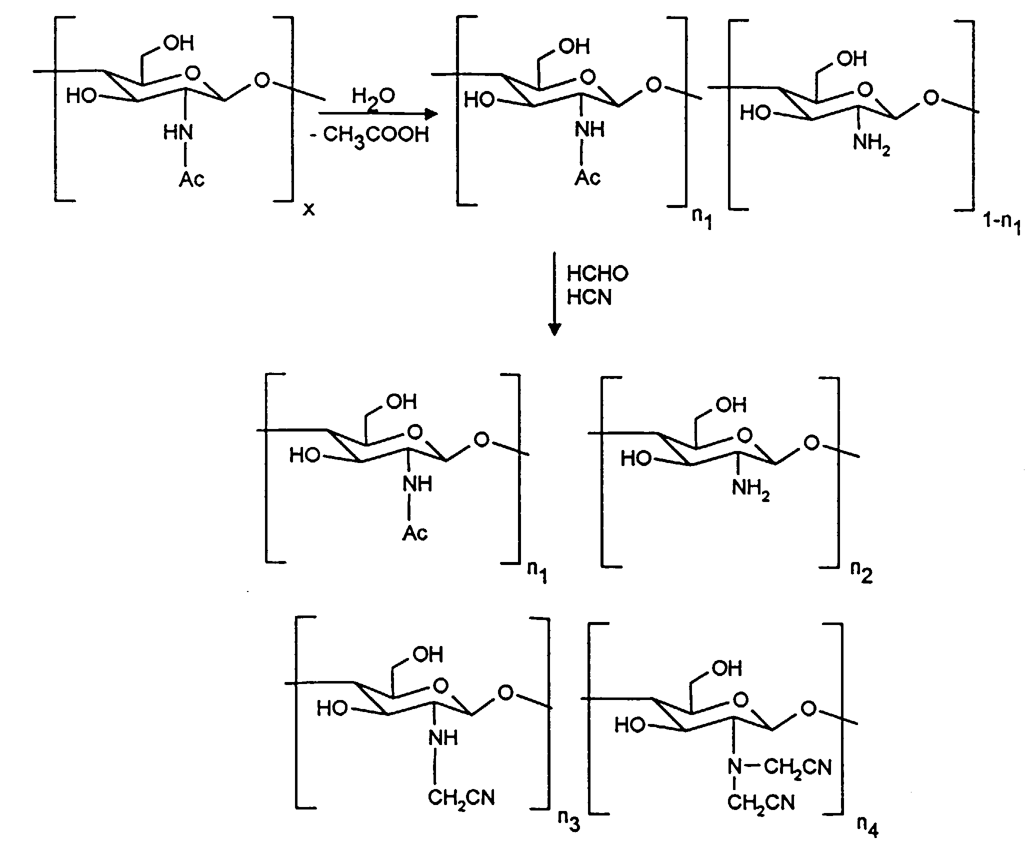 worin
- x: 5 bis 10'000, vorzugsweise 20 bis 500;
- n₁: 0 bis 0,6, vorzugsweise 0,05 bis 0,2 Teile, bezogen auf x;
- n₂: 0 bis 0,2, vorzugsweise 0,05 bis 0,1 Teile, bezogen auf x;
- (n₃ + n₄): 0,4 bis 1,0, vorzugsweise 0,8 bis 0,95 Teile, bezogen auf x;
bedeuten.

Die Umsetzung kann besonders vorteilhaft bei Temperaturen von 10 bis 70°C durchgeführt werden, vorzugsweise unter Verwendung von Ameisensäure als Lösungsmittel. Man verfährt dabei vorzugsweise so, dass man ein wasserlösliches Salz von Cyanwasserstoff, vorzugsweise NaCN, bei einer Temperatur von 0 bis 5°C in fester Form in die ameisensure Lösung des Chitosans einträgt und bei gleicher Temperatur die erste Hälfte des Formaldehyds, vorzugsweise als 37%ige-Formalin-Lösung oder Paraformaldehyd, zugibt. Anschliessend wird die Temperatur auf bis zu 70°C erhöht, sodann die zweite Hälfte des Formaldehyds zugegeben.

Die Reaktionszeiten reichen von 0,5 bis 12, vorzugsweise 2 bis 5 Stunden. Die Produktlösung wird anschliessend in eine ausreichende Menge Wasser ausgetragen. Das polymere Nitril ist bereits ab einem Substitutionsgrad von 50% vollständig wasserunlöslich und kann als bläulichgraue, zähgummi-artige Masse quantitativ isoliert werden, z.B. durch Einfällen der ameisensauren Lösung in Wasser und Nachwaschen des gefällten Polymers mit deionisiertem Wasser. Das polymere Material wird üblicherweise im Mixer in Gegenwart von Wasser zerkleinert, die Suspension mit NaOH neutralisiert, das Material abgenutscht, nachgewaschen und luftgetrocknet (Feuchtware).

Reaktionstemperatur und Reaktiondsauer haben einen wesentlichen Einfluss auf den Methylierungsgrad der N-Cyanomethylierung.

Die Herstellung der erfindungsgemässen N-cyanomethylierten Chitosane stellt einen weiteren Erfindungsgegenstand dar.

Die erfindungsgemässen Chitosanderivate sind Polymere mit filmbildenden Eigenschaften. Aus ameisensaurer Lösung bilden sich zähe, mechanisch und chemisch widerstandsfähige Filme. Sie eignen sich daher insbesondere zur Herstellung von Trennmembranen hoher Festigkeit.

Die Hydrolysate der N-cyanomethylierten Chitosane weisen wiederkehrende Einheiten der Formel 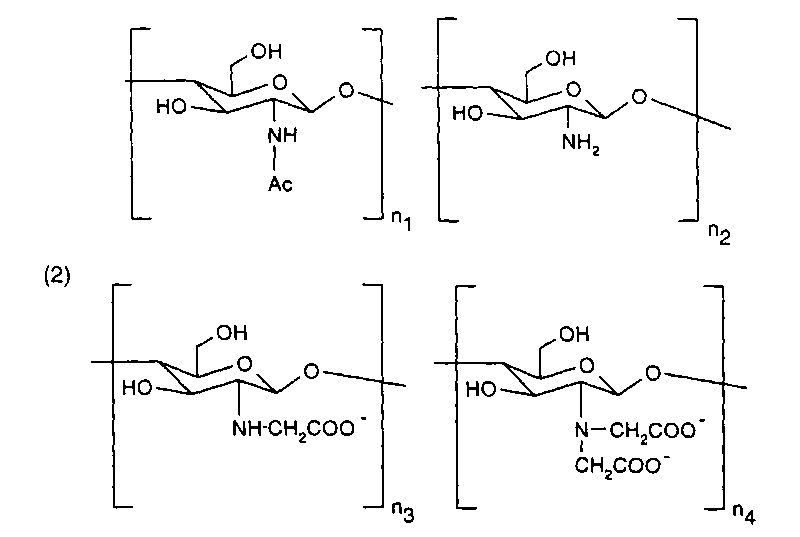 auf, worin
- n₁: 0 bis 0,6, vorzugsweise 0,05 bis 0,20; bezogen auf x;
- n₂: 0,01 bis 0,20, vorzugsweise 0,05 bis 0,10; bezogen auf x;
- (n₃ + n₄): 0,4 bis 1,0, vorzugsweise 0,8 bis 0,95; bezogen auf x;
bedeuten,
wobei x den Polymerisationsgrad von Glucosamin-Resten des Chitosans darstellt und eine Zahl von 5 bis 10000 bedeutet.

Die Herstellung der Hydrolysate aus den entsprechenden N-cyanomethylierten Verbindungen erfolgt nach folgendem Reaktionsschema: 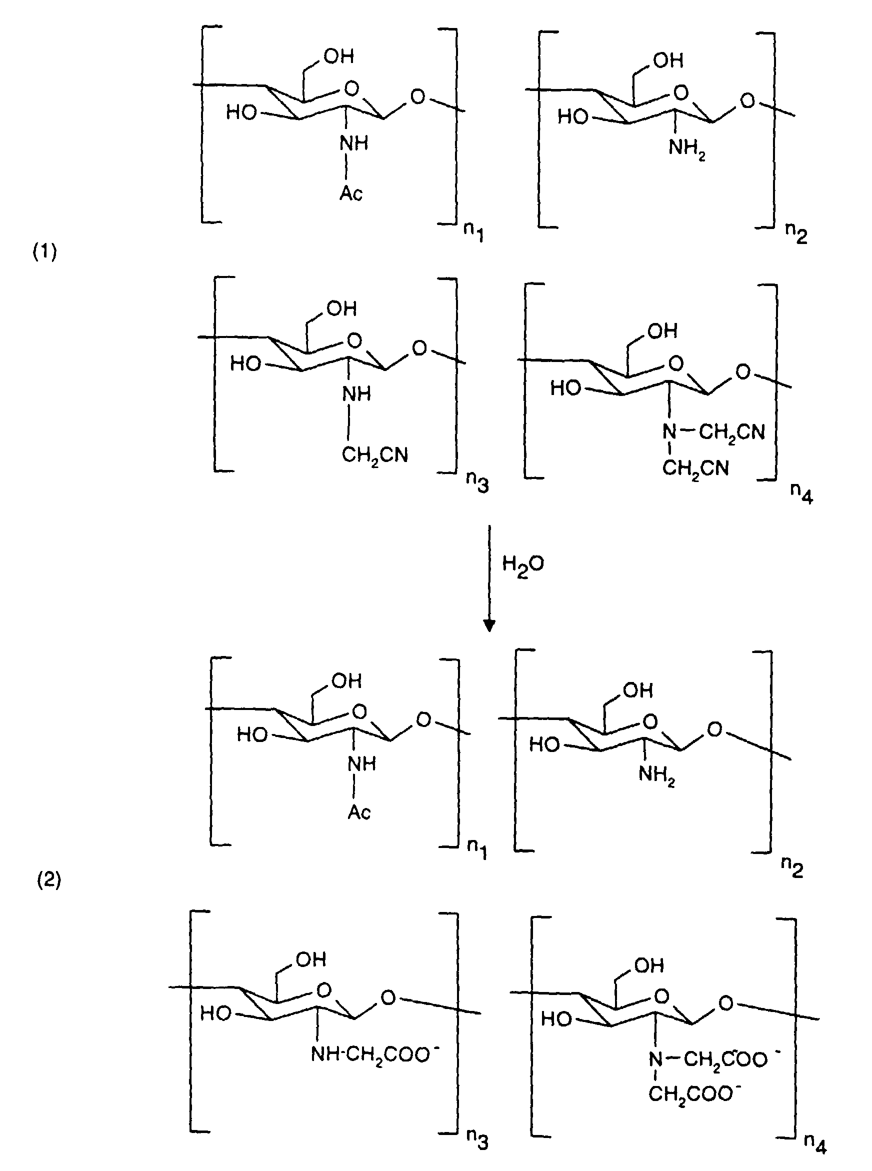 worin
- n₁: 0 bis 0,6, vorzugsweise 0,05 bis 0,20; bezogen auf x;
- n₂: 0 bis 0,20, vorzugsweise 0,05 bis 0,10; bezogen auf x;
- (n₃ + n₄): 0,4 bis 1,0, vorzugsweise 0,8 bis 0,95; bezogen auf x;
bedeuten,
wobei x den Polymerisationsgrad von Glucosamin-Resten des Chitosans darstellt und eine Zahl von 5 bis 10000 bedeutet.

Das Verfahren zur Herstellung der hydrolysierten N-cynomethylierten Chitosane stellt einen weiteren Erfindungsgegenstand dar.

Zur Hydrolyse wird die oben hergestellte Feuchtware in verdünnter Natronlauge (ca. 1,5%ig) suspendiert und ca. 50 Stunden bei ca. 80°C verkocht. Es entstehen hellgelbe, wässrige Lösungen, die auf übliche Art von Verunreinigungen befreit werden. Nach Trocknung und Zerkleinerung im Mixer erhält man ein hellgelbes Pulver.

Die hydrolysierten N-cyanomethylierten Chitosane entsprechend Formel (2) besitzen sequestrierende Eigenschaften. Auf Grund ihrer ausgeprägten sequestrierenden Wirkung für Schwermetallionen bei schon geringen Anwendungskonzentrationen lassen sich die Verseifungsprodukte zur Entfernung solcher Kationen aus kontaminiertem Wasser verwenden, zum Beispiel zur Entfernung von Eisen- oder Kupferionen aus Leitungswasser. Ferner können sie als Zusätze in zahlreichen Prozessen der Textilveredelung eingesetzt werden. Alleine oder in Kombination mit kationischen, anionischen oder neutralen Detergentien können sie als Additive in Waschmittelformulierungen dienen. Besonders hervorzuheben ist hier ihre Wirkung als Wasserenthärter und Inkrustierungsinhibitor. Ein weiterer Gegenstand der vorliegenden Erfindung ist daher die Verwendung der erfindungsgemässen hydrolysierten N-cyanomethylierten Chitosane zur Verhinderung der Haftung und/oder Bildung von festen Belägen auf anorganischen oder organischen Substraten. Bei den Substraten kann es sich um anorganische und/oder organische Beläge handeln, zum Beispiel Glas, Keramiken, Metallen und Metallegierungen, natürliche oder synthetische Kunststoffe, Papier, Textilien, Leder, oder pflanzliche oder tierische Organe oder Gewebe.

Die Hydrolysate der N-cyanomethylierten Chitosane weisen eine überraschend gute komplexierende Wirkung für Metallionen, insbesondere Erdalkalimetallionen auf, wodurch die Ausfällung von polymeren Metallsalzen, besonders bei mehrwertigen Kationen, günstig beeinflusst oder verhindert werden kann. Sie eignen sich daher auch als Zusätze in Waschmitteln, da sie zur Erhöhung der Waschkraft beitragen und Ablagerungen von abgelöstem Schmutz auf dem gewaschenen Gewebe verhindern. Darüber hinaus wirken sie als Kristallisationsverzögerer insbesondere bei der Bildung von Erdalkalicarbonaten im Waschprozess und beeinflussen so Wachstum und Morphologie der gebildeten Fällungen, deren Grössenverteilung sowie über diese Aggregation- und Adhäsionseigenschaften. Als Scale-lnhibitoren eignen sie sich daher zur Wasserbehandlung und verhindern die Abscheidung von Belägen in wasserführenden Systemen, wie z.B. Wasserbehandlungsanlagen, Leitungsrohren und auf Heizwendeln von Dampferzeugern. Im Bereich der Körperpfle geindustrie können sie u.a. als Zusätze in Zahnputzmitteln zur Verhinderung der Bildung von Zahnbelägen dienen. Sie können auch zur Vorbehandlung von Textilien, zum Beispiel Baumwolle verwendet werden.

Weiterhin finden die erfindungsgemässen hydrolysierten N-cyanomethylierten Chitosane Anwendung als Bleichmittelstabilisatoren, beispielsweise in Waschmitteln oder bei der Bleiche von Textilien, Zellstoff oder Papierrohstoff. Die eingesetzten Komplexbildner binden dabei die in den Bleichflotten vorhandenen Calcium-, Magnesium-, Eisen-, Kupfer- oder Manganionen und verhindern gleichzeitig die Ausfällung von Erdalkalicarbonat oder - hydroxid und die Zersetzung der Perverbindung.

Einen weiteren Erfindungsgegenstand bildet daher das Applikationsverfahren zum Bleichen von Cellulose enthaltenden Fasermaterialien. Das Verfahren ist dadurch gekennzeichnet, dass man das Fasermaterial mit einer wässrigen Flotte behandelt, welche mindestens eine erfindungsgemässe hydrolysierte N-cyanomethylierte Chitosanverbindung, ein Alkalimetallhydroxid, ein wasserlösliches Magnesiumsalz und eine Perverbindung enthält.

Als Perverbindung kommen z.B. Alkalimetallperoxidisulfate oder vor allem Alkalimetallperoxisulfate, wobei das Kalium- und insbesondere das Natriumperoxidisulfat oder -peroxisulfat bevorzugt sind, in Betracht. Hierbei ist das Natriumperoxisulfat (Na₂S₂O₈) ganz besonders bevorzugt, das in der Regel als Feststoff eingesetzt wird. Als Perverbindung steht Wasserstoffperoxid im Vordergrund des Interesses, das auf Grund seiner höheren Stabilität in der Regel als konzentrierte, etwa 30 bis 60 gewichtsprozentige Lösung eingesetzt wird.

Als Alkalimetallhydroxide kommen vor allem Kalium- oder insbesondere Natriumhydroxid, vorzugsweise als konzentrierte, etwa 30 gewichtsprozentige Lösung oder als festes Ätzkali oder vor allem Ätznatron, in Betracht.

Die Bleichflotten enthalten neben der erfindungsgemässen Zusammensetzung, Alkalimetallhydroxid und einer Perverbindung als fakultative Komponenten gegebenenfalls auch Netz- bzw. Waschmittel, Entschäumungs- bzw. Entlüftungsmittel und/oder optische Aufheller.

Das Applikationsverfahren zum Bleichen von Cellulose enthaltenden Fasermaterialien unter Verwendung der erfindungsgemässen Bleichmittelzusammensetzung wird nach an sich bekannten Methoden durchgeführt.

Das zu behandelnde cellulosehaltige Fasermaterial kann in verschiedenen Verarbeitungsformen vorliegen, z.B. als loses Material, Garn, Gewebe oder Gewirke. Hierbei handelt es sich in der Regel stets um textile Fasermaterialien, die aus reinen textilen Cellulosefasern oder aus Gemischen von textilen Cellulosefasem mit textilen Synthesefasern hergestellt werden.

Die erfindungsgemässen hydrolysierten N-cyanomethylierten Chitosane in Form ihrer Salze, insbesondere ihrer Erdalkali- und Ammoniumslaze sind ausserordentlich hygroskopisch und eignen sich aufgrund ihrer wasserspeichemden Wirkung als Feuchthaltemittel für die Haut oder der Schleimhäute in kosmetischen Präparaten, wie zum Beispiel in Haut- und Haarpflegemitteln und Deodoranten. Die erfindungsgemässen hydrolysierten N-cyanomethylierten Chitosane sind dabei in der Lage, Eisenionen auf der Haut zu binden und können daher die in Cremes, insbesondere Sonnencremes, üblicherweise eingesetzte EDTA ersetzen.

Einen weiteren Erfindungsgegenstand bildet daher ein kosmetisches Präparat enthaltend mindestens eine erfindungsgemässe hydrolysierte N-cyanomethylierte Verbindung, die wiederkehrende Einheiten der Formel (2) aufweist sowie kosmetisch verträgliche Träger- oder Hilfsstoffe.

Die erfindungsgemässe kosmetische Zusammensetzung enthält 0,1 bis 15, vorzugsweise 0,2 bis 10 Gew.-%, bezogen auf das Gesamtgewicht der Zusammensetzung, einer erfindungsgemässen hydrolysierten N-cyanomethylierten Chitosanverbindung, die wiederkehrende Einheiten der Formel (2) aufweist sowie kosmetisch verträglichen Hilfsstoffe.

Die kosmetische Zusammensetzung kann durch physikalisches Mischen der erfindungsgemässen hydrolysierten N-cyanomethylierten Chitosane mit den Hilfsstoffen durch gewöhnliche Methoden, wie z.B. durch einfaches Zusammenrühren der Einzelkomponenten, erfolgen.

Die erfindungsgemässe kosmetische Zusammensetzung kann als Wasser-in-Öl- oder Öl-in-Wasser-Emulsion, als Öl-Alkohol-Lotion, als vesikulare Dispersion eines ionischen oder nichtionischen amphiphilen Lipids, als Gel, fester Stift oder als Aerosol-Formulierung formuliert werden.

Für die erfindungsgemässe kosmetische Zusammensetzung kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die erfindungsgemässe kosmetische Zusammensetzung kann auch weitere Komponenten wie z.B. Emollients, Emulsionsstabilisatoren, Hautbräunungsbeschleuniger, Feuchtigkeit-Retentionsmittel, wie z.B. Glycerin, Konservierungsmittel, Duft- und Farbstoffe enthalten.

Die erfindungsgemässen hydrolysierten N-cyanomethylierten Chitosane weisen in wässrigen Lösungen auch eine viskositätssteigemde und dispergierende Wirkung auf, wodurch sie sich z.B. als Verdickungsmittel in Suspensionen, Emulsionen und wässrigen Lösungen eignen, zum Beispiel bei der Herstellung von Lebensmitteln oder Wirkstoffkonzentraten sowie Farbstoff- und Pigmentzubereitungen.

Die erfindungsgemässen hydrolysierten N-cyanomethylierten Chitosane können auch biocide Wirkungen, zum Beispiel bakteriostatische, fungistatische oder algicide Wirkungen besitzen.

Weiterhin lassen sich die erfindungsgemässen hydrolysierten N-cyanomethylierten Chitosane als Repulsionsmittel verwenden.

In den folgenden Beispielen beziehen sich Prozent und Teile stets auf das Gewicht.

### Herstellung der erfindungsgemässen N-cyanomethylierten Chitosanverbindungen

Beispiel 1: 20,0 g Chitosan (Fluka Nr. 2271, niedermolekular, durchschnittliches Molekulargewicht ca. 70,000) mit einem Wassergehalt von 9,24 %, einer Aminzahl von 4,78/43,79 mAequ/g, einer hieraus berechneten Zusammensetzung aus 11,86 mol% N-Acetyl-D-glucopyranosamin- und 88,14 mol% D-Glucopyranosamin-Einheiten, einer mittleren Mmol-masse von 166,16 und folgenden Analysendaten:

| | C | H | N | H₂O | ΔO |
|---|---|---|---|---|---|
| gefunden | 40,7 | 7,3 | 7,5 | 9,24 | 35,26 |
| gefunden, bezogen auf wasserfreie Ware | 44,8 | 6,91 | 8,26 | | 39,99 |
| berechnet | 45,09 | 6,82 | 8,43 | | 39,66 |

entsprechend 18,15 g Chitosan 100% (0,1092 mol) werden in einem mit Ankerrührer, Thermometer, Rückflusskühler mit Sperrflüssigkeitsaufsatz und Pulverdosiertrichter ausgestatteten Planschliffkolben von 800 ml Inhalt und 400 ml techn. Ameisensäure (85%) gelöst. In die nahezu klare Lösung (ungelöster Anteil < 0,5%) des Polyaminopolysaccharids werden bei 15°C über den Dosiertrichter langsam 12,5 g (0,25 mol) techn. NaCN (98 %) eingetragen und anschliessend 3 Stunden langsam ausgerührt. Bei gleicher Temperatur tropft man sodann 20,5 g Formalin 37% (7,6 g CH₂O = 0,253 Mol), mit deionisiertem Wasser auf 130 ml verdünnt, über einen Zeitraum von 1,5 Stunden zu. Anschliessend wird 24 Stunden bei Raumtemperatur nachgerührt. Die tiefblau gefärbte, hochviskose Lösung wird in 3 Liter deionisiertes Wasser ausgetragen, wo sich die Reaktionsmasse nach einiger Zeit verfestigt. Man tauscht das Fällbad gegen frisches Wasser aus und zerkleinert das grob angefallene Material im Mixer. Die erhaltene Suspension wird abgenutscht, das Nutschgut mit 3 Liter deionisiertem Wasser nachgewaschen und anschliessend bei 50°C/15 mbar über 48 Stunden getrocknet. Man erhält 26,4 g eines harten, grünblauen, spröden Materials mit folgenden Analysedaten:

| | C | H | N | H₂O | ΔO |
|---|---|---|---|---|---|
| gefunden | 48,6 | 5,3 | 14,2 | 0,31*) | 31,59 |
| gefunden, bezogen auf wasserfreie Ware | 48,7 | 5,28 | 14,24 g | | 31,73 |

| | | | | | |
|---|---|---|---|---|---|
| *) ausgetrieben bei 130°C. | | | | | |

Der aus den Analysedaten berechnete Substitutionsgrad DS ergibt sich zu 0,725. Die hieraus berechneten Analysedaten sind:

| | C | H | N | O |
|---|---|---|---|---|
| berechnet | 48,49 | 5,94 | 13,93 | 31,63 |

Das N-cyanomethylierte Polyaminopolysaccharid ist löslich in DMSO und konzentrierter Ameisensäure. Die Zusammensetzung wird durch das lR-Spektrum bestätigt (Absorptionsbande bei 2249 cm⁻¹ für -N-CH₂CN; Banden bei 1673 cm⁻¹ und 1553 cm⁻¹ für die N-Acetylgruppe).

Beispiel 2: Ein Labor-Schaufeltrockner mit einem Volumen von 1000 ml, 2 Dossieröffnungen sowie Thermostatanschluss wird mit 800 ml techn. Ameisensäure (85%) geladen. Anschliessend werden bei laufendem Rührwerk nach und nach 40 g des in Beispiel 1 beschriebenen Chitosans eingetragen. Man rührt 15 Stunden bei Raumtemperatur bis zur praktisch vollständigen Lösung des Materials. Nach Erreichen einer über den Thermostaten geregelten Innentemperatur von 15°C werden über die beiden Dossieröffnungen gleichzeitig langsam je 18,5 g technisches NaCN (98%) (98 %; = 36,26 g; 100 % = 0,74 mol) zudosiert. Anschliessend rührt man 3 Stunden bei 15°C aus, erwärmt dann auf Raumtemperatur und tropft jetzt sehr langsam und unter guter Umwälzung eine Lösung von 40,5 g Formalin 37% (= 15 g CH₂O = 0,5 mol), mit dest. Wasser auf ein Gesamtvolumen von 100 ml verdünnt, und hälftig auf 2 Tropftrichter verteilt, zu. Nach Zugabe von jeweils 30 ml der Formalinlösung wird die Innentemperatur über den Thermostaten auf 40°C erhöht und bei dieser Temperatur die restliche Formalinlösung zugetropft. Anschliessend rührt man 20 Stunden bei 40°C nach. Man erhält eine hochviskose, tief grünblau gefärbte Lösung des N-cyanomethylierten Polyaminopolysaccharids, die direkt in fünf Liter deionisiertes Wasser ausgetragen wird, wo sich das Reaktionsprodukt allmählich als grünblaue Masse verfestigt. Das Fällbad wird dekantiert, die Polymermasse in frischem Wasser aufgenommen und anschliessend im Mixer zerkleinert. Die erhaltene Suspension wird zur Abtrennung eingeschlossener Salze 24 Stunden ausgerührt, die wässrige Phase sodann mit 30%iger NaOH auf einen pH-Wert von 7,5 eingestellt, wobei das Reaktionsprodukt eine Farbaufhellung erfährt (Umschlag von grünblau auf dunkelgelb). Die Suspension wird abgenutscht und mit fünf Liter deionisiertem Wasser nachgewaschen.
Ausbeute (feucht): 469,7 g.

29,9 g der feuchten Rohware werden bei 50°C/15 mbar über 48 Stunden getrocknet und ergeben 3,3 g einer gelb-hellbraunen, harten, spröden Masse mit folgenden Analysedaten:

| | C | H | N | H₂O | ΔO |
|---|---|---|---|---|---|
| gefunden | 47,7 | 5,6 | 14,3 | 0,83*) | 31,57 |
| gefunden, bezogen auf wasserfreie Ware | 48,1 | 5,56 | 14,42 | | 31,92 |

| | | | | | |
|---|---|---|---|---|---|
| *) ausgetrieben bei 150°C | | | | | |

Der aus den Analysedaten berechnete Substitutionsgrad DS ergibt sich zu 0,745. Die hieraus berechneten Analysedaten sind

| | C | H | N | O |
|---|---|---|---|---|
| berechnet | 48,59 | 5,92 | 14,10 | 31,41 |

Das Produkt ist in DMSO und konz. Ameisensäure löslich. Die Zusammensetzung wird durch das IR-Spektrum bestätigt (Absorptionsbande bei 2249 cm⁻¹ für -N-CH₂CN, Banden bei 1673 cm⁻¹ für die N-Acetylgruppe).

Beispiel 3: 20 g des in Beispiel 1 beschriebenen Chitosans werden in einem mit Ankerrührer, Thermometer, Rückflusskühler mit Sperrflüssigkeitsaufsatz und Pulver-Dossiertrichter ausgestatteten Planschliffkolben von 800 ml Inhalt in 400 ml techn. Ameisensäure (85 %) gelöst. In die nahezu klare Lösung (ungelöster Anteil < 0,5 %) des Polyaminopolysaccharids werden bei Raumtemperatur langsam 22,2 g Formalin 37% (= 8,2 g CH₂O = 0,273 mol) eingetropft und 30 Minuten lang ausgerührt. Sodann wird innerhalb von 1,5 Stunden und ebenfalls bei Raumtemperatur eine Lösung von 6,4 g (0,128 mol) techn. NaCN (98 %) in 20 ml dest. Wasser zugetropft. Man rührt 5 Stunden aus, erhöht die Temperatur auf 50°C und tropft erneut eine Lösung von 7,7 g (0,154 mol) techn. NaCN (98 %) in 30 ml dest. Wasser ein. Es wird 24 Stunden bei 50°C und 3 Stunden bei 60°C nachgerührt, die tief grünblau gefärbte, hochviskose Lösung sodann in 3 Liter deionisiertes Wasser ausgeladen, wo sich die Reaktionsmasse nach und nach verfestigt. Nach Abdekantieren des Fällbades und Aufnahme in frischem Wasser wird die Polymermasse im Mixer zerkleinert, die erhaltene Suspension mit 30%iger NaOH auf pH 7,5 eingestellt. Man saugt ab und erhält 174,4 g eines feuchten Nutschgutes, von dem 43,6 g im Trockenschrank bei 50°C/15 mbar über 12 Stunden getrocknet werden. Die Rückwaage ergibt 6,4 g einer gelbbraunen, harten, spröden Masse mit folgenden Analysedaten:

| | C | H | N | H₂O | ΔO |
|---|---|---|---|---|---|
| gefunden | 46,9 | 5,6 | 12,7 | 1,94*) | 32,86 |
| gefunden, bezogen auf wasserfreie Ware | 47,83 | 5,49 | 12,95 | | 33,73 |

| | | | | | |
|---|---|---|---|---|---|
| *) bestimmt durch Austreiben bei 130°C | | | | | |

Der aus den Analysedaten berechnete Substitutionsgrad DS ergibt sich zu 0,565. Die hieraus berechneten Analysedaten sind

| | C | H | N | O |
|---|---|---|---|---|
| berechnet | 47,74 | 6,14 | 12,72 | 33,40 |

Das Produkt ist in konz. Ameisensäure löslich. Das lR-Spektrum stimmt qualitativ mit den lR-Spektren aus den Beispielen 1 und 2 überein. Molmasse: 198,4

Beispiel 4: In einem mit Ankerrührer, Rückflusskühler und Thermometer ausgestatteten Planschliffkolben von 800 ml Inhalt werden 130 g der in Beispiel 3 erhaltenen Feuchtware (ca. 8,7 g Trockenmasse =0,0934 mol) eingewogen, anschliessend 200 ml deionisiertes Wasser und 120 ml IN NaOH zugesetzt Man rührt 48 Stunden bei einer Badtempemtur von 100°C. Gasförmig entweichender Ammoniak wird mit pH-Papier nachgewiesen. Man trägt in zwei Liter deionisiertes Wasser aus und klärt die nach mehrstündigem Ausrühren erhaltene gelbbraune, geringe Gelanteile enthaltene Lösung über Filtergaze 140 S. Das unter vermindertem Druck (30 mbar/50°C) auf ein Volumen von ca. 100 ml eingeengte Filtrat (stark viskose, klare Lösung) wird in 3 Liter EtOH_{abs} eingefällt, das ausgefallene polyaminopolycarbonsaure Na-Salz abgenutscht und bei 50°C/0,5 mbar über 48 Stunden getrocknet.
Ausbeute: 21,8 g.
Filterrückstand: 2,8 g (ca. 8 %).

Das Produkt ist mit einem pH-Wert von 10 klar löslich in Wasser.

Aminzahl: 3,64/3,70 mAequ/g (bestimmt durch Rücktitration mit 0,1 N NaOH nach Zugabe eines Überschusses an Salzsäure; Titrationsdauer: 1,5 Stunden).
Summe Amin- + Carboxyzahl: 6,06/6,16 mAequ/g (bestimmt durch Titration mit 0,1 N HClO₄ in H₂O/Eisessig 1:30; nach 2 bzw. 16 Stunden Rühren)
Carboxyzahl: 2,44 mAequ/g (aus Differenz)
Das Produkt ist ausserordentlich hygroskopisch.

| Löslichkeit als Funktion des pH-Wertes: | |
|---|---|
| Konzentration [g/l] | beginnende Ausfällung (bei pH) |
| 9,13 | 5,2 |
| 4,28 | 4,3 |
| 1,06 | 3,2 |

Die erhaltene Polyaminopolycarbonsäure lässt sich somit als inneres Salz ausfällen und abtrennen.
Ca²⁺⁻Sequestrierkapazität: 102 mg Ca²⁺/g
lR-Spektrum: Nitrilbande und Banden für N-Acetygruppe sind verschwunden

Beispiele 5 bis 11: In diesen Beispielen werden verschiedene Chitosane in verschiedenen Mengen eingesetzt unter Variation des Verhältnisses Cyanwasserstoff/Formaldehyd. Die verwendeten Chitosane (= C1-C4) sind in Tabelle 1 aufgeführt.

**Tabelle 1:**

| eingesetztes Chitosan | | | | | | | | | | |
|---|---|---|---|---|---|---|---|---|---|---|
| | | | | | | | Flememtaranalyse¹ | | | |
| | (Chi tin)ₓ x | (Chito san)_{y} x | H₂O Gehalt [%]² | Aminzahl [mEq/g]³ | Deacet.-Grad [mol%] | MG [Da] | C_{ber}/ C_{gef} | H_{ber}/ H_{gef} | N_{ber}/ N_{gef} | O_{ber}/ O_{gef} |
| | [mol%] | [mol%] | | | | | | | | |
| C1 | 0,1231 | 0,8769 | 9,24 | 5,2720 | 87,69 | 166,355 | 45,10/ 44,84 | 6,82 6,91 | 8,42 8,26 | 39,66 39,66 |
| C2 | 0,1333 | 0,8667 | 11,20 | 5,1971 | 86,67 | 166,765 | 45,13/ 45,12 | 6,81 6,75 | 8,40 8,13 | 39,66 40,00 |
| C3 | 0,1383 | 0,8617 | 11,45 | 5,1609 | 86,17 | 166,974 | 45,15/ 45,25 | 6,81 6,84 | 8,39 8,20 | 39,65 39,71 |
| C4 | 0,0930 | 0,9070 | 7,42 | 5,4946 | 90,70 | 165,071 | 45,01/ 44,93 | 6,83 6,91 | 8,49 8,40 | 39,67 39,76 |

| | | | | | | | | | | |
|---|---|---|---|---|---|---|---|---|---|---|
| ¹ berechnet auf wasserfreie Ware; Berechnungsgrundlage: Aminzahl | | | | | | | | | | |
| ² bestimmt durch Austreiben bei 130°C | | | | | | | | | | |
| ³ Einwaage mit exakt 0,5 ml 0,1 N HCl + 3 ml dest. Wasser versetzt; nach 4 Tagen titriert mit 0,1 N | | | | | | | | | | |

In der Tabelle 2a sind die Reaktionsbedingungen der Umsetzungen sowie die Zusammensetzung der erhaltenen Chitosan-N-essigsäurenitrile angegeben.

**Tabelle 2a:**

| Beisp. | Eingesetztes Chitosan | Einw. [g]⁴ | Lösungsmittel [ml][%] | NaCN/CH₂O | (Chitin)ₓ | (Chitosan)_{y} | (Chitosan-DN)_{z}⁵ | DS(%) | Ausbeute (g)⁶ | Geh. FW (%) | MG (Da) |
|---|---|---|---|---|---|---|---|---|---|---|---|
| 5 | C1 | 40,0 | 800 | 0,7399/0,5015 | 0,1231 | 0,2069 | 0,6700 | 76,4 | 407,90 | 12,71 | 218,6 |
| | | | 85 | | | | | | | | |
| 6 | C1 | 40,0 | 900 | 0,7399/0,5015 | 0,1231 | 0,2693 | 0,6076 | 69,3 | 511,64 | 10,52 | 213,8 |
| | | | 98 | | | | | | | | |
| 7 | C2 | 40,0 | 800 | 0,7399/0,5015 | 0,1333 | 0,2646 | 0,6021 | 69,5 | 522,16 | 9,58 | 213,8 |
| | | | 85 | | | | | | | | |
| 8 | C3 | 36,6 | 500 | 0,4295/0,4302⁷ | 0,1383 | 0,3613 | 0,5000 | 58,0 | 49,57 | 97,92 | 206,0 |
| | | | 98 | | | | | | | | |
| 9 | C4 | 43,2 | 1100 | 0,7320/0,6000 | 0,0930 | 0,3000 | 0,6070 | 66,9 | 505,2 | 12,90 | 212,5 |
| | | | 85 | | | | | | | | |
| 10 | C4 | 43,2 | 1200 | 0,7320/0,6000 | 0,0930 | 0,2770 | 0,6300 | 69,5 | 415,8 | 14,51 | 214,3 |
| | | | 85 | | | | | | | | |
| 11 | C4 | 43,2 | 1200 | 0,7320/0,6100 | 0,0930 | 0,2700 | 0,6370 | 70,2 | 474,2 | 13,17 | 214,8 |
| | | | 85 | | | | | | | | |

| | | | | | | | | | | | |
|---|---|---|---|---|---|---|---|---|---|---|---|
| Natronlauge ⁴ Einwaage Substanz tel quel | | | | | | | | | | | |
| ⁵ Chitos. DN= Chitosan-N-diessigsäurenitril | | | | | | | | | | | |
| ⁶ Ausbeute Feuchtware (FW), luftgetrocknet (Ausnahme Beispiel 8; getrocknet bei 60°C im Hochvakuum | | | | | | | | | | | |
| ⁷ CH₂O eingesetzt als Paraformaldehyd; gesamte Reaktion bei Raumtemperatur | | | | | | | | | | | |

**Tablle 2b:**

| Elementaranalyse⁸ | | | | | | | | |
|---|---|---|---|---|---|---|---|---|
| Beispiel | | | | | | | | |
| 5 | C_{ber} | 49,04 | H_{ber} | 5,8 | N_{ber} | 14,99 | O_{ber} | 30,17 |
| | C_{gef} | 49,44 | H_{gef} | 5,2 | N_{gef} | 14,76 | O_{gef} | 30,6 |
| | | | | | | | | |
| 6 | C_{ber} | 48,75 | H_{ber} | 5,88 | N_{ber} | 14,51 | O_{ber} | 30,86 |
| | C_{gef} | 49,08 | H_{gef} | 5,62 | N_{gef} | 14,36 | O_{gef} | 30,94 |
| | | | | | | | | |
| 7 | C_{ber} | 48,74 | H_{ber} | 5,88 | N_{ber} | 14,44 | O_{ber} | 30,94 |
| | C_{gef} | 49.37 | H_{gef} | 5,21 | N_{gef} | 14,13 | O_{gef} | 31,29 |
| | | | | | | | | |
| 8 | C_{ber} | 48,26 | H_{ber} | 6,01 | N_{ber} | 13,60 | O_{ber} | 32,14 |
| | C_{gef} | 48,51 | H_{gef} | 5,07 | N_{gef} | 13,503 | O_{gef} | 32,91 |
| | | | | | | | | |
| 9 | C_{ber} | 48,70 | H_{ber} | 5,88 | N_{ber} | 14,60 | O_{ber} | 30,82 |
| | C_{gef} | 48,87 | H_{gef} | 5,17 | N_{gef} | 14,54 | O_{gef} | 31,42 |
| | | | | | | | | |
| 10 | C_{ber} | 48,81 | H_{ber} | 5,86 | N_{ber} | 14,77 | O_{ber} | 30,57 |
| | C_{gef} | 48,92 | H_{gef} | 5,23 | N_{gef} | 14,99 | O_{gef} | 31,36 |
| | | | | | | | | |
| 11 | C_{ber} | 48,84 | | 5,85 | N_{ber} | 14,83 | O_{ber} | 30,49 |
| | C_{gef} | 49,03 | | 5,03 | N_{gef} | 14,40 | O_{gef} | 31,54 |

| | | | | | | | | |
|---|---|---|---|---|---|---|---|---|
| ⁸ Wassergehaltsbestimmung: H₂O bei 130°C ausgetrieben (K: Fischer) | | | | | | | | |

Die Umsetzung erfolgt dabei folgendermassen:
NaCN in fester Form wird bei einer Temperatur von 0 bis 5°C langsam in die ameisensaure Lösung des partial deacetylierten Chitins (C1-C4) eingetragen und bei gleicher Temperatur die erste Hälfte des Formaldehyds (als 37%ige Formalin-Lösung oder Paraformaldehyd) zugegeben. Anschliessend wird die Temperatur auf 40°C (resp. 60°C) erhöht, sodann die zweite Hälfte des Formaldehyds zugegeben. Nach Ausreagieren wird die Produktlösung (meist blaugrünlich gefärbt) in eine ausreichende Menge Wasser ausgetragen. Das polymere Nitril ("Chitosan"-N-(di)essigsäurenitril) ist bereits ab Substitutionsgrad 50% vollständig wasserunlöslich und kann als bläulichgraue, zähe, gummiartige Masse quantitativ isoliert werden. Das Verhältnis Fällbad zu Reaktionslösung beträgt dabei ca. 5:1. Das polymere Material wird im Mixer in Gegenwart von Wasser zerkleinert, die Suspension mit 4 N NaOH neutralisiert, das Material, das noch Ameisensäure inkludiert enthält, abgenutscht, nachgewaschen und luftgetrocknet (= Feuchtware). Das Material wird zur Verseifung (siehe Beispiele 12 bis 18) in diesem gequollenen Zustand eingesetzt.

Von der Feuchtware wird ein Muster von ca. 10g genau eingewogen, dieses bei 60° über 24 Stunden am Hochvakuum getrocknet, die Gewichtsdifferenz (Wasser) bestimmt, ein Muster zur Elementaranalyse gegeben, das Wasser auf das getrocknete Muster umgerechnet, zum Differenzwasser addiert und die Gesamtwassermenge in der Feuchtware auf diese hochgerechnet. Die Differenz ist die wasserfreie Ausbeute.

Zur vereinfachten Berechnung des Substitutionsgrades (DS) wird auf ein Terpolymer mit den Einheiten Chitin (Anteil des Ausgangsmaterials, bleibt unter den Reaktionsbedingungen unverändert), Chitosan und Chitosan-N-di-esssigsäurenitril bezogen.

### Beispiele 12 bis 18:

Die in den Beispielen 5 bis 11 erhaltenen erhaltenen Chitosan-N-essigsäurenitrile werden zur Verseifung als Feuchtware (FW) in ca. 1,5%iger Natronlauge suspendiert und ausreichend lange bei 80°C verkocht. Die entstandenen hellgelben, wässrigen Lösungen werden über eine G 2-Sinterfritte geklärt, am Rotationsverdampfer bei 60-70°C (Wasserstrahlvakuum) eingeengt, die hochviskosen Lösungen sodann in Ethanol (6%ig) eingefällt. Das abgenutschte Material wird bei 50°C im Trockenschrank über CaCl₂ im Hochvakuum getrocknet und anschliessend im Mixer zerkleinert.
Aspekt: hellgelbes , fast farbloses Pulver. Die polymere Aminosäure mit dem Polysaccharid-Backbone enthält Na-Formiat als Beimengung, das beim Einfällen in Ethanol mit ausgefällt wird.

In der Tabelle 3a sind die Reaktionsparameter wowie die Zusammensetzungen der Verseifungsprodukte aufgeführt.

**Tabelle 3a:**

| | | | | | | Zusammensetzung | | | | | |
|---|---|---|---|---|---|---|---|---|---|---|---|
| Beispiel | FW [g] | H₂O deion .[ml] | NaOH [mol] | Reakt. Zeit [h] | Ausbeute⁹ [g] [%] | (Chitosan)ₓ x[mol] | (Chitos.-ND-Na)_{y} y [mol] | (HCOONa)_{z} z [mol] | MG [Da] | IEP¹⁰ [pH] | η_{red}¹¹ [dl/g] [%G/V] |
| 12¹² | 157,53 | 650 | 0,364 | 38 | 27,21 93,1 | 0,3300 | 0,6700 | 0,7500 | 319,4 | 5,24 | 6,576 0,5009 |
| 13¹³ | 190,02 | 650 | 0,330 | 72 | 25,32 85,8 | 0,3924 | 0,6076 | 0,8400 | 315,6 | 4,71 | 9,014 0,5020 |
| 14¹⁴ | 208,86 | 650 | 0,329 | 48 | 26,58 96,4 | 0,3979 | 0,6021 | 0,5420 | 294,4 | 4,73 | 2,730 0,5007 |
| 15¹⁵ | 20,42 | 830 | 0,295 | 96 | 22,23 74,1 | 0,5000 | 0,5000 | 1,0000 | 309,2 | 4,60 | 3,970 0,5008 |
| 16¹⁶ | 494,70 | 2368 | 1,120 | 120 | 90,99 94,4 | 0,3930 | 0,6070 | 0,9190 | 320,8 | - | 6,861 0,5008 |
| 17¹⁷ | 403,60 | 2224 | 1,028 | 120 | 88,9 98,9 | 0,3700 | 0,6300 | 0,9800 | 328,6 | - | 4,174 |
| 18¹⁸ | 464,60 | 2268 | 1,069 | 120 | 87,6 92,8 | 0,3630 | 0,6370 | 1,0900 | 337,2 | - | 2,977 0,5013 |

| | | | | | | | | | | | |
|---|---|---|---|---|---|---|---|---|---|---|---|
| ⁹ bezogen auf wasserfreie Ware | | | | | | | | | | | |
| ¹⁰ IEP= isoelektrischer Punkt; alkalimetrisch bestimmt nach R.A.A. Muzzarelli et al., Carbohydrate Research 107, 202, 207 und 208 (1982) | | | | | | | | | | | |
| ¹¹ reduzierte Viskosität; gemessen mit Ubbelohde-Visokosimeter Typ I, 25°C, Lösungen der angegebenen Konzentration [%G/V] in ention. Wasser | | | | | | | | | | | |
| ¹² Chitosan-N-Essigsäurenitril aus Beispiel 5 | | | | | | | | | | | |
| ¹³ Chitosan-N-Essigsäurenitril aus Beispiel 6 | | | | | | | | | | | |
| ¹⁴ Chitosan-N-Essigsäurenitril aus Beispiel 7 | | | | | | | | | | | |
| ¹⁵ Chitosan-N-Essigsäurenitril aus Beispiel 8 | | | | | | | | | | | |
| ¹⁶ Chitosan-N-Essigsäurenitril aus Beispiel 9 | | | | | | | | | | | |
| ¹⁷ Chitosan-N-Essigsäurenitril aus Beispiel 10 | | | | | | | | | | | |
| ¹⁸ Chitosan-N-Essigsäurenitril aus Beispiel 11 | | | | | | | | | | | |

**Tabelle 3b:**

| Elementaranalyse¹⁹ | | | | | | | | | | |
|---|---|---|---|---|---|---|---|---|---|---|
| Beispiel | | | | | | | | | | |
| 12 | C_{ber} | 35,46 | H_{ber} | 4,13 | N_{ber} | 4,39 | O_{ber} | 40,98 | Na_{ber} | 15,04 |
| | C_{gef} | 34,25 | H_{gef} | 4,07 | N_{gef} | 4,28 | O_{gef} | 42,35 | Na_{gef} | 15,05 |
| | | | | | | | | | | |
| 13 | C_{ber} | 35,29 | H_{ber} | 4,17 | N_{ber} | 4,44 | O_{ber} | 41,13 | Na_{ber} | 14,97 |
| | C_{gef} | 33,61 | H_{gef} | 4,00 | N_{gef} | 4,20 | O_{gef} | 43,26 | Na_{gef} | 14,93 |
| | | | | | | | | | | |
| 14 | C_{ber} | 36,52 | H_{ber} | 4,36 | N_{ber} | 4,76 | O_{ber} | 40,72 | Na_{ber} | 13,64 |
| | C_{gef} | 36,74 | H_{gef} | 4,71 | N_{gef} | 4,16 | O_{gef} | 40,73 | Na_{gef} | 13,68 |
| | | | | | | | | | | |
| 15 | C_{ber} | 34,96 | H_{ber} | 4,24 | N_{ber} | 4,53 | O_{ber} | 41,40 | Na_{ber} | 14,87 |
| | C_{gef} | 34,48 | H_{gef} | 4,05 | N_{gef} | 4,41 | O_{gef} | 42,25 | Na_{gef} | 14,81 |
| | | | | | | | | | | |
| 16 | C_{ber} | 34,99 | H_{ber} | 4,13 | N_{ber} | 4,37 | O_{ber} | 41,23 | Na_{ber} | 15,29 |
| | C_{gef} | 33,89 | H_{gef} | 4,81 | N_{gef} | 3,68 | O_{gef} | 42,82 | Na_{gef} | 14,80 |
| | | | | | | | | | | |
| 17 | C_{ber} | 34,72 | H_{ber} | 4,06 | N_{ber} | 4,26 | O_{ber} | 41,26 | Na_{ber} | 15,67 |
| | C_{gef} | 33,90 | H_{gef} | 4,69 | N_{gef} | 3,79 | O_{gef} | 42,60 | Na_{gef} | 15,02 |
| | | | | | | | | | | |
| 18 | C_{ber} | 34,33 | | 4,00 | N_{ber} | 4,15 | O_{ber} | 41,41 | Na_{ber} | 16,12 |
| | C_{gef} | 33,07 | | 4,36 | N_{gef} | 3,55 | O_{gef} | 42,94 | Na_{gef} | 16,08 |

| | | | | | | | | | | |
|---|---|---|---|---|---|---|---|---|---|---|
| ¹⁹ Wassergehaltsbestimmung: H₂O bei 130°C ausgetrieben (K: Fischer) | | | | | | | | | | |

### Beispiel 19: Bestimmung des Schwermetall- und Ca-Komplexiervermögens der in der Tabelle 4a beschriebenen Polysaccharid-poplyaminocarbonsäuren

**Tabelle 4:**

| | | | | | |
|---|---|---|---|---|---|
| Polysaccharid-poplyaminocarbonsäuren aus Beispiel | Fe³⁺ [mg/g): pH 6 | Ni²⁺ [mg/g): pH 6 | Cd²⁺ [mg/g): pH 6 | Mn²⁺ [mg/g): pH 6 | Ca²⁺ [mg/g): pH 9,5 |
| 12 | 117 | 52 | 109 | 76 | 65 |
| 13 | 134 | 66 | 118 | 69 | 63 |
| 14 | 125 | 50 | 104 | 66 | 67 |
| 15 | 132 | 57 | 110 | 68 | 66 |
| 16 | 122 | 80 | 99 | 93 | 96 |
| 17 | 113 | 75 | 89 | 80 | 88 |
| 18 | 136 | 69 | 101 | 95 | 85 |

### Beispiel 19: Anwendung in der Kosmetik

Herstellung einer Sonnenschutzcreme:

| | | | |
|---|---|---|---|
| 7,5 T | Octylmethoxyzinnamat | 1,0 T | PEG-40-Stearat |
| 5 T | Octylsalicylat | 1,0 T | Methicon |
| 5 T | Methylanthranilat | 0,75 T | Cetylalkohol |
| 2 T | Isocetylalkohol | 0,25 T | Tocopherylacetat und |
| 2 T | Cetearylalkohol | 6 T | Zinkoxid |
| 1,5 T | Glycerylstearat | | |

werden gemischt und auf 75 bis 80°C erhitzt Anschliessend dispergiert man mit einer handelsüblichen Kugel-, Vibrations- oder Hammermühle
0,5 T Magnesium-aluminiumsilikat in
63,8 T Wasser.
Der Dispersion gibt man sodann
0,5 T Xanthan-Gummi
und mischt, bis eine homogene Lösung entsteht. Unter Zugabe von
0,2 T der in Beispiel 4 hergestellten Verbindung
wird auf 75 bis 80°C erhitzt. Zu dieser Lösung gibt man unter Rühren die anfangs gemischten und auf 75 bis 80°C erhitzten Verbindungen. Man mahlt 10 bis 15 Minuten, kühlt auf 40°C ab und gibt
1 T Propylenglykol
hinzu. Unter weiterem Rühren lässt man auf Raumtemperatur abkühlen.

Die Zubereitung eignet sich als feuchtigkeitsspendende Sonnenschutzcreme.

## Patentansprüche

1. N-cyanomethylierte Chitosane, enthaltend wiederkehrende Einheiten der Formel 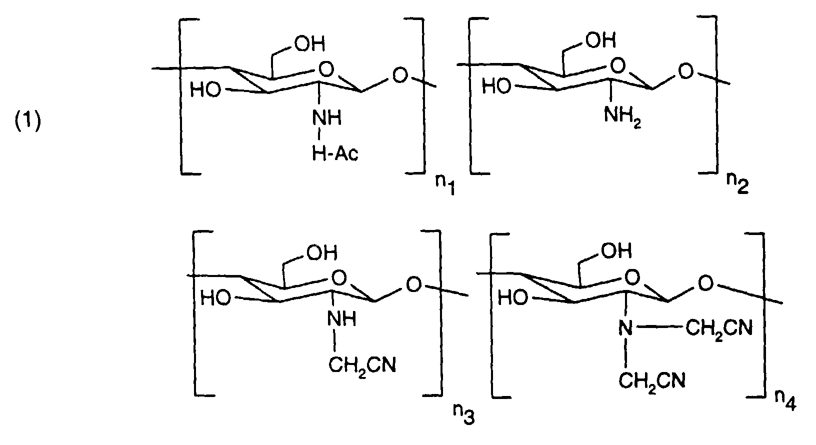 worin
n₁ 0 bis 0,6 Teile, bezogen auf x;
n₂ 0 bis 0,20 Teile, bezogen auf x;
(n₃ + n₄) 0,4 bis 1,0 Teile, bezogen auf x;
bedeuten,
wobei x den Polymerisationsgrad von Glucosamin-Resten des Chitosans darstellt und eine Zahl von 5 bis 10000 bedeutet.

2. Verfahren zur Herstellung der N-cyanomethylierten Chitosane nach Anspruch 1, dadurch gekennzeichnet, dass man Chitosan mit Formaldehyd und Cyanwasserstoff bei einer Temperatur von 10 bis 70 °C und einer Reaktionszeit von 0,5 bis 12, vorzugsweise 2 bis 5 Stunden nach folgendem Reaktionsschema 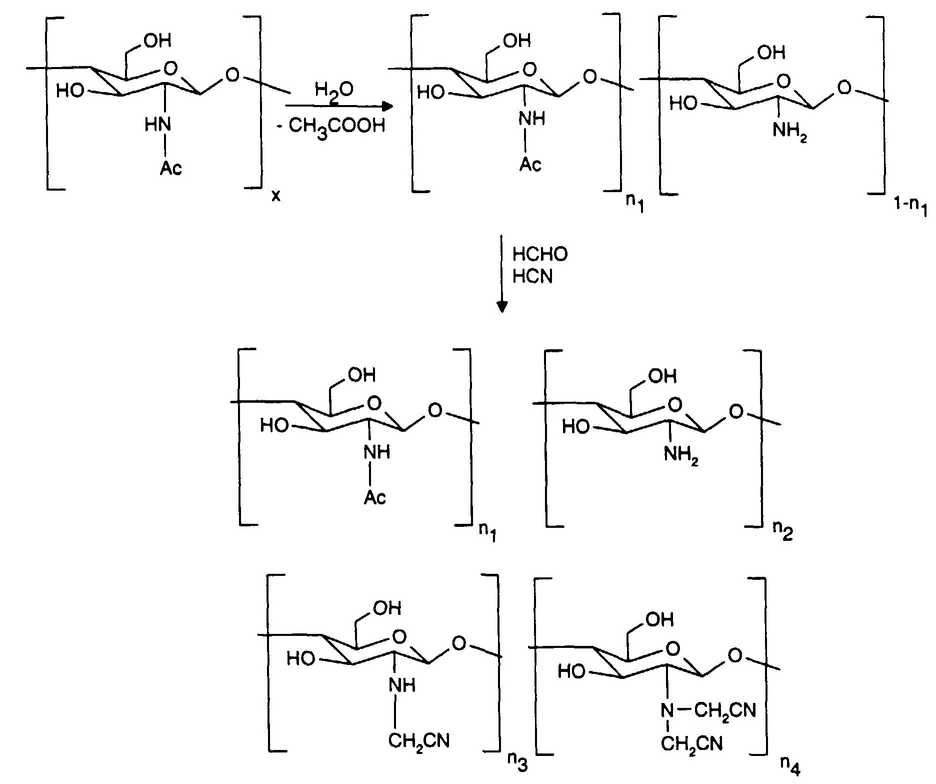 worin
x 5 bis 10'000;
n₁ 0 bis 0,6, bezogen auf x;
n₂ 0 bis 0,2, bezogen auf x;
(n₃ + n₄) 0,4 bis 1,0, bezogen auf x;
bedeuten,
umsetzt.

3. Verfahren nach Anspruch 2, dadurch gekennzeichnet, dass die Reaktion in Anwesenheit von Ameisensäure durchgeführt wird.

4. Verwendung der N-cyanomethylierten Chitosane nach Anspruch 1 als Trennmembranen.

5. Verfahren zur Herstellung der hydrolysierten N-cyanomethylierten Chitosane der Formel (2), dadurch gekennzeichnet, dass man die N-cyanomethylierten Chitosane enthaltend wiederkehrende Einheiten der Formel (1) nach folgendem Schema zu den hydrolysierten N-cyanomethylierten Chitosanen der Formel (2) 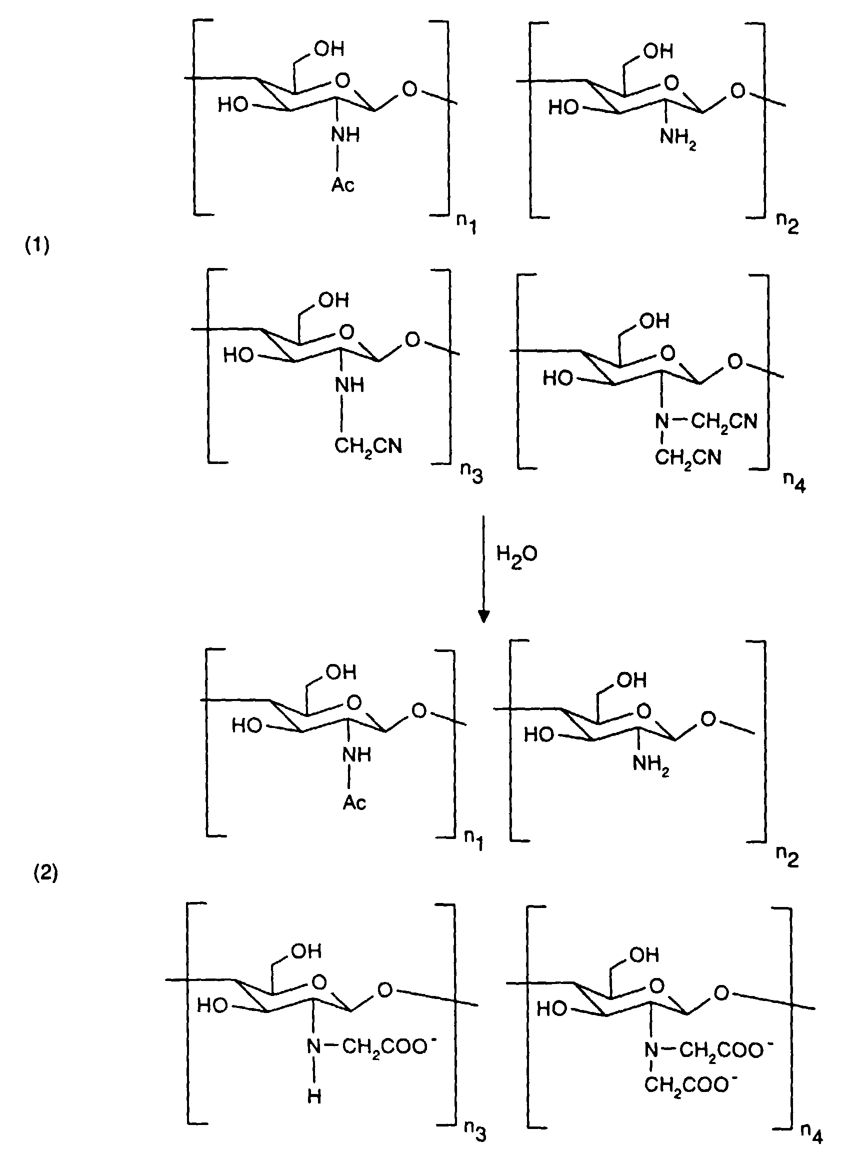 worin
n₁ 0 bis 0,6, bezogen auf x;
n₂ 0 bis 0,20, bezogen auf x;
(n₃ + n₄) 0,4 bis 1,0, bezogen auf x;
bedeuten, hydrolysiert.
wobei x den Polymerisationsgrad von Glucosamin-Resten des Chitosans darstellt und eine Zahl von 5 bis 10000 bedeutet.

6. Verwendung der hydrolysierten N-cyanomethylierten Chitosane nach Anspruch 5 als Sequestriermittel für Erdalkalimetallionen, Komplexbildner für Metallionen oder Stabilisator für Bleichmittel.

7. Verwendung der hydrolysierten N-cyanomethylierten Chitosane nach Anspruch 5 zur Verhinderung von Haftung und/oder Bildung von festen Belägen auf anorganischen oder organischen Substraten.

8. Kosmetisches Präparat enthaltend mindestens ein hydrolysiertes N-cyanomethyliertes Chitosan nach Anspruch 5 sowie kosmetisch verträgliche Träger- oder Hilfsstoffe.

9. Verwendung der hydrolysierten N-cyanomethylierten Chitosane nach Anspruch 5 als Feuchthaltemittel der Haut und von Schleimhäuten.

10. Verwendung der hydrolysierten N-cyanomethylierten Chitosane nach Anspruch 5 als Repulsionsmittel.

11. Verwendung der hydrolysierten N-cyanomethylierten Chitosane nach Anspruch 5 als Verdickungsmittel.

12. Verwendung der hydrolysierten N-cyanomethylierten Chitosane nach Anspruch 5 als Repulsionsmittel.

## Claims

1. An N-cyanomethylated chitosan comprising repeating units of formula 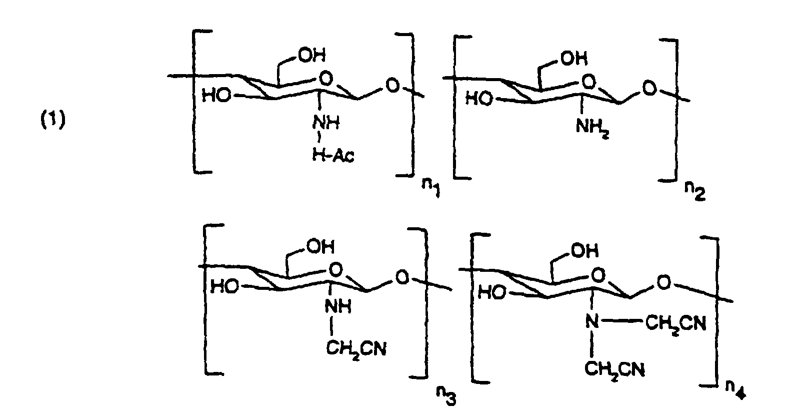 wherein
n₁ is 0 to 0.6 parts, based on x;
n₂ is 0 to 0.20 parts, based on x;
(n₃+n₄) is 0.4 to 1.0 parts, based on x;
representing the degree of polymerisation of glucosamine residues of the chitosan and being a number from 5 to 10,000.

2. A process for the preparation of an N-cyanomethylated chitosan according to claim 1, which comprises reacting chitosan with formaldehyde and hydrogen cyanide at a temperature from 10 to 70°C and in a reaction time of from 0.5 to 12 hours, preferably from 2 to 5 hours, in accordance with the following reaction scheme: 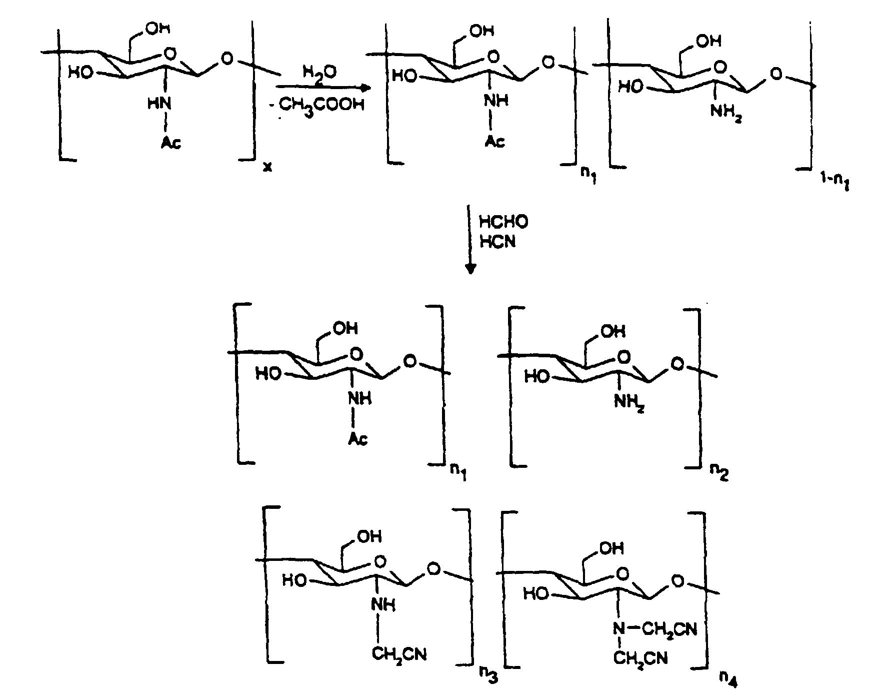 wherein
x is from 5 to 10,000;
n₁ is 0 to 0.6, based on x;
n₂ is 0 to 0.2, based on x;
(n₃+n₄) is 0.4 to 1.0, based on x;

3. A process according to claim 2, wherein the reaction is carried out in the presence of formic acid.

4. The use of an N-cyanomethylated chitosan according to claim 1 as a separating membrane.

5. A process for the preparation of the hydrolysed N-cyanomethylated chitosan of formula (2), which comprises hydrolysing an N-cyanomethylated chitosan comprising repeating units of formula (1) according to the following scheme to a hydrolysed N-cyanomethylated chitosan of formula (2) 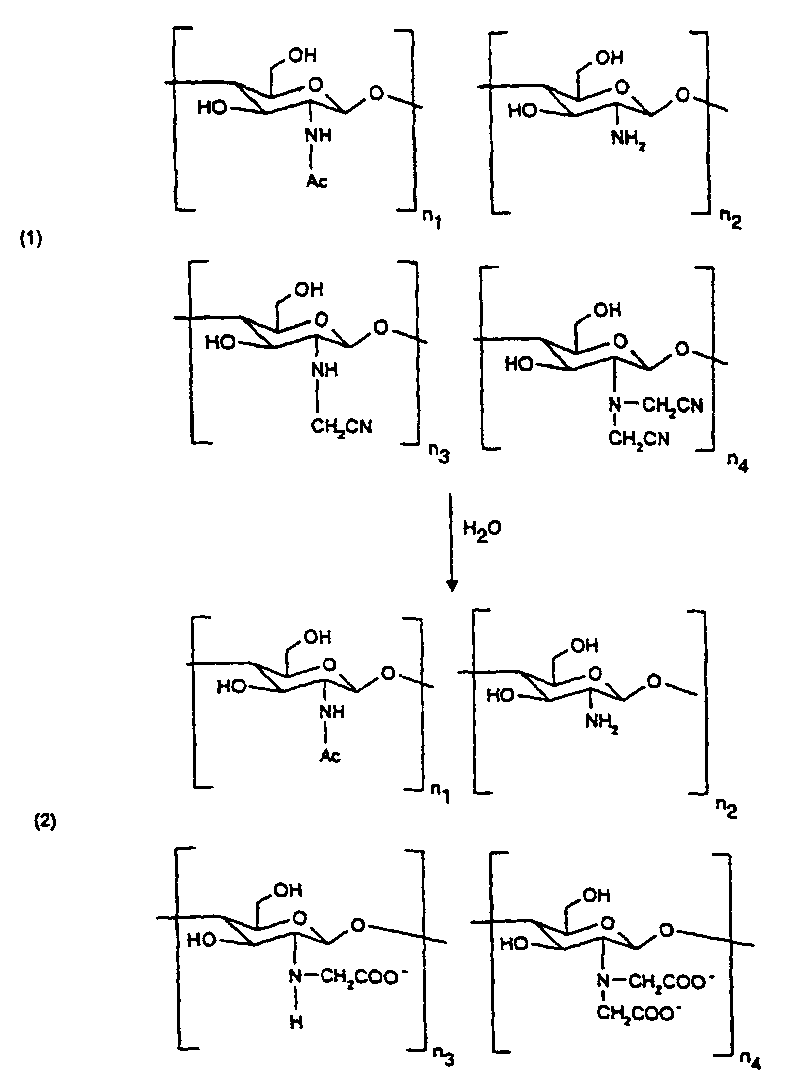 wherein
n₁ is 0 to 0.6, based on x;
n₂ is 0 to 0.20, based on x;
(n₃+n₄) is 0.4 to 1.0, based on x;
representing the degree of polymerisation of glucosamine residues of the chitosan and being a number from 5 to 10,000.

6. The use of a hydrolysed N-cyanomethylated chitosan according to claim 5 as a sequestrant for alkaline earth metal ions, complexing agent for metal ions, or stabiliser for bleaches.

7. The use of a hydrolysed N-cyanomethylated chitosan according to claim 5 for preventing the adhesion and/or the formation of solid deposits to or on organic or inorganic substrates.

8. A cosmetic composition comprising at least one hydrolysed N-cyanomethylated chitosan according to claim 5, together with cosmetically acceptable carriers or adjuvants.

9. The use of a hydrolysed N-cyanomethylated chitosan according to claim 5 as a moisture retainer for the skin and mucous membranes.

10. The use of a hydrolysed N-cyanomethylated chitosan according to claim 5 as a repellant.

11. The use of a hydrolysed N-cyanomethylated chitosan according to claim 5 as a thickener.

12. The use of a hydrolysed N-cyanomethylated chitosan according to claim 5 as a repellant.

## Revendications

1. Chitosanes N-cyanométhylés contenant des motifs répétitifs de formule 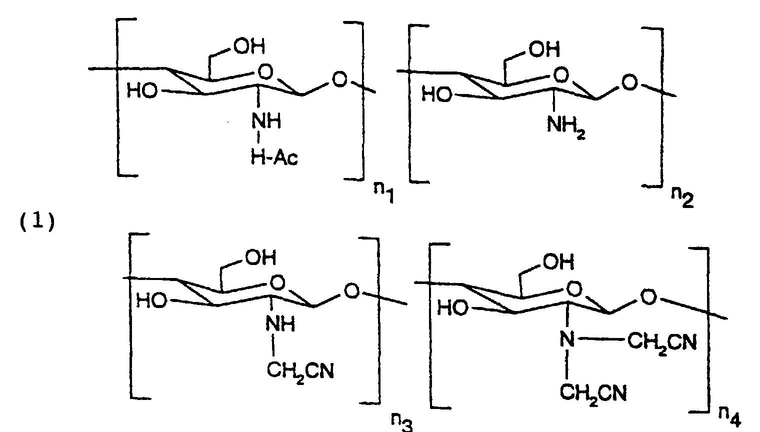 où
n₁ va de 0 à 0,6 parties, par rapport à x ;
n₂ va de 0 à 0,20 parties, par rapport à x ;
(n₃ + n₄) va de 0,4 à 1,0 parties, par rapport à x ;
x étant le degré de polymérisation des restes glucosamine des chitosanes et représente un nombre de 5 à 10000.

2. Procédé pour la préparation des chitosanes N-cyanométhylés selon la revendication 1, caractérisé en ce qu'on fait réagir le chitosane sur le formaldéhyde et l'acide cyanhydrique à une température de 10 à 70°C et pendant une durée réactionnelle de 0,5 à 12, de préférence de 2 à 5 heures selon le schéma réactionnel suivant 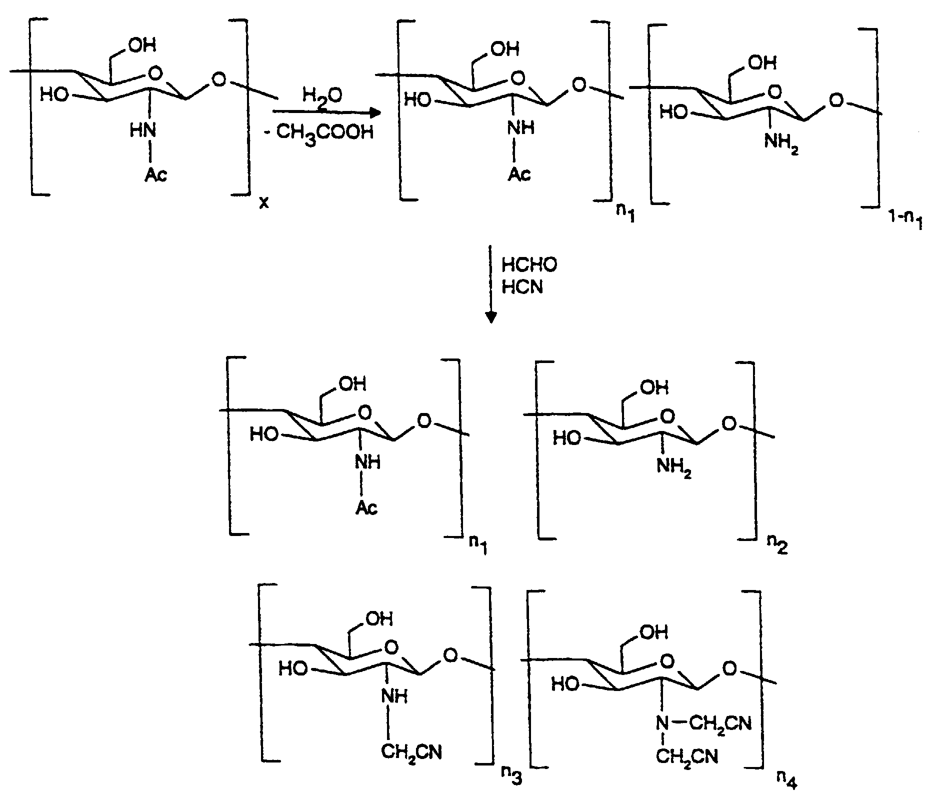
x va de 5 à 10000 ;
n₁ va de 0 à 0,6, par rapport à x ;
n₂ va de 0 à 0,2, par rapport à x ;
(n₃ + n₄) va de 0,4 à 1,0 par rapport à x.

3. Procédé selon la revendication 2, caractérisé en ce que la réaction est mise en oeuvre en présence de l'acide formique.

4. Utilisation des chitosanes N-cyanométhylés selon la revendication 1 comme membranes de séparation.

5. Procédé de préparation des chitosanes N-cyanométhylés hydrolysés de formule (2), caractérisé en ce qu'on hydrolyse les chitosanes N-cyanométhylés contenant des motifs répétitifs de formule (1) selon le schéma suivant pour obtenir les chitosanes N-cyanométhylés de formule (2) 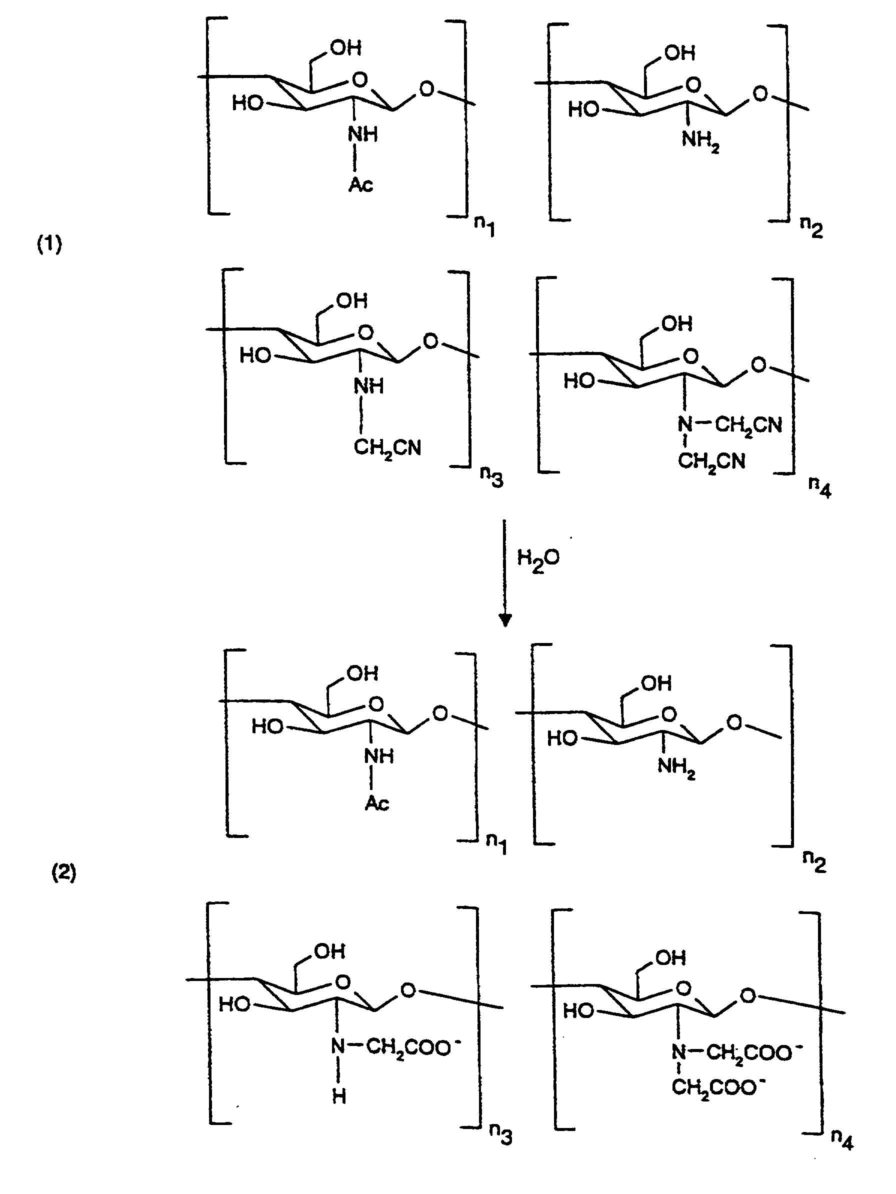 où
n₁ va de 0 à 0,6, par rapport à x ;
n₂ va de 0 à 0,20, par rapport à x ;
(n₃ + n₄) va de 0,4 à 1,0, par rapport à x ;
x étant le degré de polymérisation des restes glucosamine du chitosane et représente un nombre de 5 à 1000.

6. Utilisation des chitosanes N-cyanométhylés hydrolysés selon la revendication 5 comme agent de séquestration d'ions de métaux alcalins, comme agents complexants d'ions métalliques ou stabilisant de produits de blanchiment.

7. Utilisation des chitosanes N-cyanométhylés hydrolysés selon la revendication 5 pour empêcher l'adhésion et/ou la formation de dépôt solides sur des substrats inorganiques ou organiques.

8. Préparation cosmétique contenant au moins un chitosane N-cyanométhylé hydrolysé selon la revendication 5 ainsi que des véhicules et adjuvants tolérés en cosmétique.

9. Utilisation des chitosanes N-cyanométhylés hydrolysés selon la revendication 5 comme produit hydratant pour la peau et les muqueuses.

10. Utilisation des chitosanes N-cyanométhylés hydrolysés selon la revendication 5 comme répulsif.

11. Utilisation des chitosanes N-cyanométhylés hydrolysés selon la revendication 5 comme épaississant.

12. Utilisation des chitosanes N-cyanométhylés hydrolysés selon la revendication 5 comme répulsif.
